(19) Europäisches Patentamt
European Patent Office
Office européen des brevets

(11) Numéro de publication : **0 336 855 B1**

(12) **FASCICULE DE BREVET EUROPEEN**

(45) Date de publication du fascicule du brevet :
**13.05.92 Bulletin 92/20**

(51) Int. Cl.⁵ : **A61M 5/315**

(21) Numéro de dépôt : **89420100.3**

(22) Date de dépôt : **20.03.89**

(54) **Seringue à usage unique.**

(30) Priorité : **21.03.88 FR 8804118**

(43) Date de publication de la demande :
**11.10.89 Bulletin 89/41**

(45) Mention de la délivrance du brevet :
**13.05.92 Bulletin 92/20**

(84) Etats contractants désignés :
**AT BE CH DE ES GB GR IT LI LU NL SE**

(56) Documents cités :
**EP-A- 0 229 017
GB-A- 2 015 883**

(56) Documents cités :
**GB-A- 2 117 249
GB-A- 2 205 750
US-A- 2 902 995
US-A- 4 699 614**

(73) Titulaire : **MICROTECHNIC S.A.
5 rue de l'Industrie
Monaco (MC)**

(72) Inventeur : **Sultan, Jean-Claude
Route de la Marteraye
F-74410 Saint Jorioz (FR)**

(74) Mandataire : **Maureau, Philippe et al
Cabinet GERMAIN & MAUREAU BP 3011
F-69392 Lyon Cédex 03 (FR)**

**Description**

La présente invention concerne une seringue à usage unique du type comprenant un corps cylindrique à l'intérieur duquel est mobile un piston délimitant, de manière étanche, avec l'extrémité du corps comportant un support d'aiguille, une chambre intérieure de volume variable, le piston étant relié à l'extrémité intérieure d'une tige de piston permettant la manoeuvre de celui-ci.

De telles seringues sont utilisées dans le domaine médical. Elles sont livrées stériles et sont destinées normalement à un usage unique afin d'éviter tout risque de contamination. Ces seringues sont généralement vendues avec leurs pistons en position de remplissage, c'est-à-dire enfoncés en bout de course à l'intérieur du corps.

Toutefois, ces seringues présentent un inconvénient majeur : leur structure technique ne permet pas d'empêcher la réutilisation d'une seringue souillée. Ce phénomène est particulièrement courant chez des populations à risques telles que les toxicomanes, où une seringue peut servir à plusieurs injections pour une même personne ou pour des personnes différentes. Par ailleurs, il n'est pas exclu que certaines réutilisations puissent se produire par inadvertance en milieu hospitalier.

Or, de telles pratiques sont particulièrement propices à la propagation de maladies infectieuses très graves telles que le S.I.D.A, ou l'hépatite B.

Pour remédier à ces inconvénients, il a été imaginé de réaliser des seringues à usage unique.

Le document EP-A-0 229 017 concerne une seringue de ce type dans laquelle la tige de piston est attelée de façon amovible au piston, afin d'assurer une désolidarisation entre la tige et le piston lorsqu'une traction est exercée sur la tige postérieurement à la première injection. A cet effet, le piston est prolongé du côté de la tige par une partie conique puis par une partie cylindrique et un disque présentant une collerette périphérique. La tige de piston est pour sa part équipée de plusieurs pattes qui, agissant sur les bords du disque à la façon d'une pince, présentent au niveau de leur liaison avec la tige des zones d'amincissement ou de rupture. En fin d'injection, les pattes prennent appui sur la partie conique et s'écartent, ce qui se traduit par leur rupture ou une déformation suffisante pour qu'elles ne puissent plus agripper le bord du disque.

Toutefois cette solution ne donne pas satisfaction. En effet, la force agissant sur les pattes d'accrochage est variable en fonction de plusieurs paramètres : la viscosité, le diamètre de l'aiguille et la vitesse d'injection. Une faible viscosité du fluide, une faible vitesse d'injection, ou un gros diamètre de l'aiguille ne génèreront pas une résistance assez importante pour entraîner la cassure des pattes pendant l'injection. La fiabilité de cette seringue est donc aléatoire. D'autre part, il est possible avant la première utilisation de démonter la seringue et de bloquer les pattes en les encerclant, par exemple à l'aide d'un ruban adhésif, ce qui rend impossible ultérieurement leur écartement et par suite leur rupture.

Le document US-A-4 669 614 concerne une seringue à usage unique dont la tige de piston est réalisée en deux parties décalées axialement et associées par des doigts radiaux de la première partie pénétrant dans des rainures d'une paroi circulaire de la seconde partie. La forme des rainures assure, après première injection, la désolidarisation des deux parties de la tige du piston de la seringue.

Le but essentiel de la présente invention est de procurer une seringue à usage unique ne permettant qu'une seule injection en interdisant de façon sûre et irrémédiable un second remplissage de la chambre intérieure de la seringue.

A cet effet, cette seringue du type comprenant un corps cylindrique creux à l'intérieur duquel est mobile un piston délimitant, de manière étanche, avec l'extrémité du corps comportant un support d'aiguille, une chambre intérieure de volume variable, le piston étant relié à l'extrémité intérieure d'une tige de piston permettant la manoeuvre de celui-ci, et des moyens permettant, d'une part, de solidariser le piston à l'extrémité intérieure de la tige de piston lors du premier remplissage de la chambre intérieure et, d'autre part, de provoquer la désolidarisation du piston et de l'extrémité intérieure de la tige de piston lors d'une éventuelle tentative de remplissage ultérieur de la chambre intérieure, est caractérisée en ce que l'extrémité intérieure de la tige de piston comporte un embout d'axe correspondant à celui du corps et présentant au moins une région tronconique dont le plus grand diamètre est situé du côté du piston, cette région tronconique étant disposée, au montage, dans un prolongement creux du piston tourné du côté de la tige, de manière à former un coin bloquant ce prolongement contre un organe de mémorisation, constitué par un anneau dont la périphérie est en appui contre la paroi du corps cylindrique.

Afin de faciliter le montage de la seringue et d'optimiser son fonctionnement, la région tronconique de l'embout de la tige est prolongée, d'une part, par une partie cylindrique fixée sur la tige de piston et, d'autre part, du côté de son extrémité libre, par une partie tronconique convergente.

Selon une caractéristique intéressante de l'invention, le prolongement creux du piston est constitué par quatre pattes s'étendant à partir de la périphérie de celui-ci, décalées angulairement de 90° les unes par rapport aux autres, comprenant chacune, depuis le piston, une partie parallèle à l'axe de la seringue prolongée par une partie inclinée vers l'intérieur, les faces extérieure et intérieure de chaque patte étant destinées à venir en contact respectivement avec la face intérieure de l'anneau de mémorisation et la région tronconique de l'embout.

Pour permettre le décalage de l'anneau de mémorisation lors de l'injection, la longueur de la partie convergente de l'embout est inférieure à celle de la partie de chaque patte du piston parallèle à l'axe de la seringue.

Avantageusement, l'embout est fixé à l'extrémité de la tige par clipsage sur un têton solidaire de celle-ci.

Lors du premier remplissage de la seringue, le prolongement du piston est coincé entre l'anneau de mémorisation de l'embout de la tige de la seringue.

La partie conique de l'embout assure donc l'entraînement du piston dans le sens de la sortie de la tige.

Lors de la réalisation de l'injection, obtenue par poussée sur la tige, il se produit un décalage entre le piston et l'anneau de mémorisation, de telle sorte que ce dernier n'enserre plus les pattes constitutives du prolongement du piston. Il en résulte que si une traction est exercée sur la tige, en vue d'un nouveau remplissage, les pattes n'étant plus retenues par l'anneau de mémorisation, laissent échapper l'embout, si bien que le piston ne peut plus être retiré.

Afin de garantir de façon encore plus sûre l'usage unique de la seringue, le support d'aiguille situé à l'une des extrémités du corps se présente avantageusement sous la forme d'un tube de diamètre inférieur à celui du corps reliant la chambre intérieure de la seringue à l'extérieur et destiné à recevoir, sur sa surface extérieure, un capuchon de forme complémentaire supportant la partie métallique creuse de l'aiguille à l'une de ses extrémités, le tube étant disposé à l'intérieur d'une jupe cylindrique solidaire du corps par l'une de ses extrémités et possèdant une longueur supérieure à celle du tube de telle sorte que, lorsque le capuchon est emmanché sur le tube, la jupe enveloppe totalement ce capuchon.

Cette disposition empêche le retrait de l'aiguille après sa mise en place sur le support du corps, car il est peu commode et dangereux de la manipuler en la saisissant par sa fine partie métallique, cette partie étant la seule préhensible.

Afin de parfaire l'inviolabilité du montage de l'aiguille, le capuchon est avantageusement équipé d'un rebord annulaire apte à s'engager, de façon irréversible, dans un logement de forme complémentaire prévu sur la jupe ou sur le tube.

De toute façon, l'invention sera mieux comprise et ses avantages ressortiront bien de la description qui suit, en référence au dessin schématique annexé représentant, à titre d'exemples non limitatifs, deux formes d'exécution de cette seringue à usage unique.

Figure 1 en est une vue en perspective éclatée,

Figure 2 en est une vue en coupe longitudinale avant le remplissage,

Figure 3 en est une vue en coupe longitudinale après le remplissage,

Figure 4 en est une vue en coupe longitudinale après l'injection,

Figure 5 en est une vue en coupe longitudinale lors d'une tentative de second remplissage,

Figure 6 est une vue partielle, en coupe longitudinale, de la partie avant d'une variante d'exécution de cette seringue.

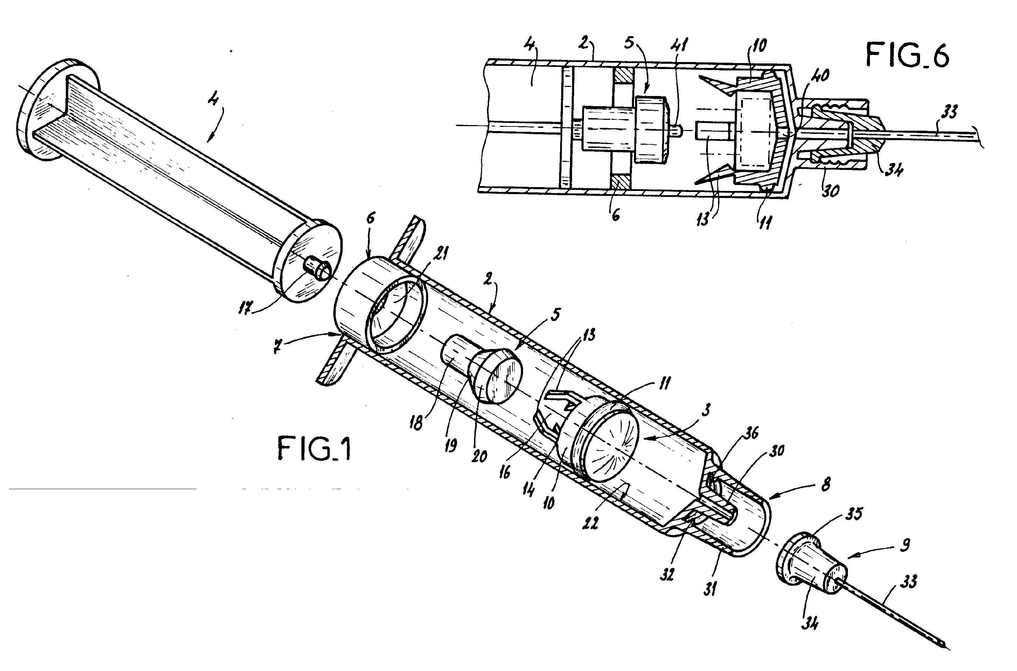

La figure 1 représente la seringue 1 selon l'invention. Elle est constituée par un corps de seringue 2 cylindrique creux, par exemple en plastique ou verre, vu en coupe longitudinale à l'intérieur duquel sont montés un piston 3, une tige de piston 4 destinée à être reliée à un embout 5 et un anneau de mémorisation 6. Ces éléments sont introduits par l'extrémité ouverte 7 du corps de la seringue 2, son autre extrémité étant conçue comme un support 8 pour une aiguille 9.

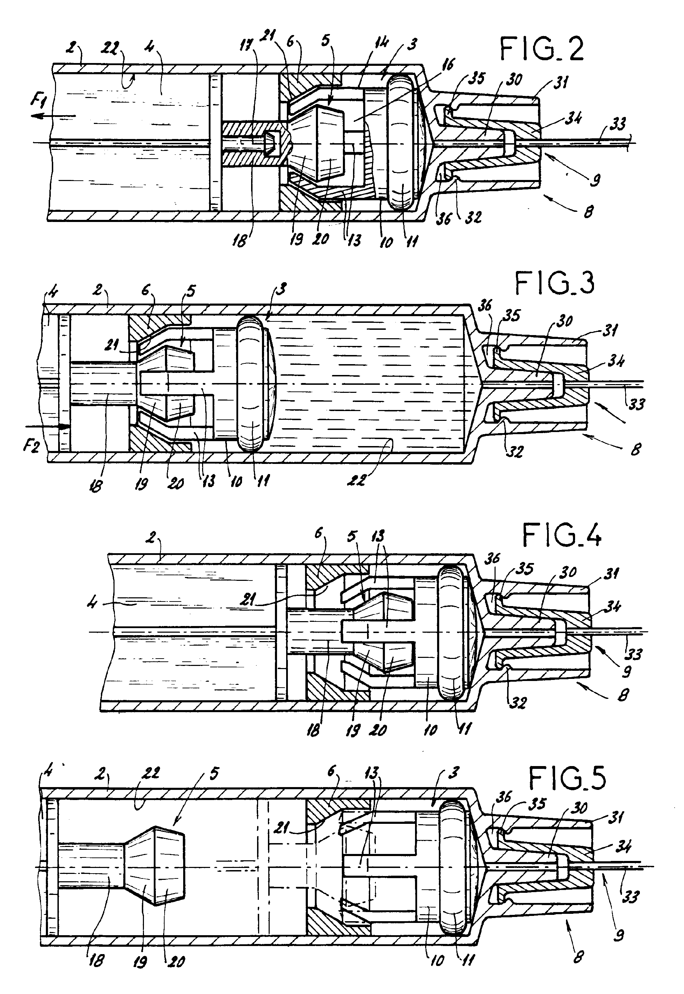

Comme le montrent également les figures 2 à 5, le piston 3 réalisé par exemple en une matière synthétique est constitué par une partie cylindrique 10 de diamètre inférieur à celui du corps 2 équipée d'un joint torique 11 fixé dans une gorge annulaire non référencée. Ce joint torique 11 permet de délimiter, avec l'extrémité 8 du corps de la seringue 2, une chambre intérieure 12 étanche.

La partie cylindrique 10 du piston se prolonge en direction de la tige de piston 4 par quatre pattes 13 identiques entre elles. Ces pattes 13 s'étendent à partir de la périphérie de la partie cylindrique 10 et sont décalées angulairement de 90° les unes par rapport aux autres. Chaque patte 13 de section rectangulaire comprend, depuis le piston 3, une partie parallèle à l'axe de la seringue 1 se terminant par une partie inclinée vers l'intérieur, la face extérieure de sa partie parallèle à l'axe de la seringue 1 étant désignée par 14 sur le dessin. A l'intérieur du prolongement creux 16 du piston 3, défini par les quatre pattes 13, est disposé l'embout 5 constituant l'extrémité intérieure de la tige de piston 4. Cet embout 5 d'axe correspondant à celui du corps 2 est fixé sur la tige 4 par l'intermédiaire d'un têton 17 solidaire de celle-ci et s'enclipsant dans une cavité de forme complémentaire ménagée dans une partie cylindrique 18 de l'embout 5. Celle-ci se prolonge par une région tronconique 19 dont le plus grand diamètre est situé du côté du piston 3 et se termine enfin par une partie tronconique convergente 20 dont la longueur est inférieure à celle de la partie parallèle à l'axe de la seringue 1 de chaque patte 13.

L'embout 5 de la tige 4 et le piston 3 sont reliés par l'intermédiaire de l'anneau de mémorisation 6.

En effet, ainsi qu'il ressort des figures 2 et 3, les faces extérieure 14 et intérieure de chaque patte 13 sont maintenues en contact respectivement avec la face intérieure 21 de l'anneau de mémorisation 6 et la région tronconique 19 de l'embout 5. La périphérie de l'anneau de mémorisation 6 est elle-même en appui contre la paroi 22 du corps 2.

L'embout 5, par l'intermédiaire de sa région tronconique **19**, est donc conçu comme un coin bloquant les pattes **13** contre l'anneau de mémorisation **6**. La face interne **21** de l'anneau **6** et les faces extérieures **14** des pattes **13** sont de forme complémentaire.

En ce qui concerne le support **8** pour l'aiguille **9**, il est constitué par un tube **30** de diamètre inférieur à celui du corps **2**. Ce tube **30** met en communication la chambre intérieure **12** avec l'extérieur. Il possède un axe correspondant à celui du corps **2** et est disposé à l'intérieur d'une jupe **31** cylindrique creuse solidaire du corps **2** par l'une de ses extrémités. Cette jupe **31** possède un diamètre et une longueur supérieurs à ceux du tube **30**. Elle présente, par ailleurs, sur la face interne de sa paroi, au voisinage du corps, une nervure annulaire **32**.

L'aiguille **9** est constituée par une partie métallique creuse **33** solidaire par l'une de ses extrémités d'un capuchon **34** en matière plastique destiné à s'emmancher sur le tube **30**. Ce capuchon **34** présente, sur le pourtour de son extrémité ouverte, un rebord annulaire **35**.

Ainsi qu'il ressort des figures 2 à 5, après emmanchement du capuchon **34** sur le tube **30**, le rebord annulaire **35** est bloqué, dans un logement **36** de la jupe **31**, par la nervure annulaire **32** empêchant ainsi le retrait de l'aiguille **9**.

Le fonctionnement de la seringue **1** selon l'invention est illustré par les figures 2 à 5.

La figure 2 représente la seringue **1**, telle que montée et prête à l'emploi. Le piston **3** est enfoncé à fond de course à l'intérieur du corps creux **2**. La chambre intérieure **12** possède ainsi un volume réduit au minimum.

Le premier et unique remplissage de cette chambre **12** s'effectue en plongeant la partie métallique fine **33** de l'aiguille **9** dans le liquide à injecter et en exerçant ensuite une traction dans le sens de la flèche F1 (figure 2) sur la tige de piston **4**.

Le piston **3**, dont les prolongements des pattes **13** sont coincés entre l'anneau de mémorisation **6** et la région tronconique **19** de l'embout **5**, est entraîné par la tige **4**. Le coincement, encore accentué par la traction sur la tige **4** des prolongements **13** entre l'embout **5** et l'anneau de mémorisation **6**, implique que ce dernier est également entraîné en translation avec le piston **3** et la tige **4**. La dépression ainsi créée amène le liquide à injecter dans la chambre intérieure **12** que l'on remplit jusqu'au volume souhaité. La figure 3 illustre cette situation.

Il ne reste plus qu'à procéder à l'injection. Pour cela, on exerce une pression sur la tige de piston **4** dans le sens de la flèche F2 (figure 3).

L'extrémité libre de l'embout **5** vient alors en contact avec le piston **3** et l'entraîne en translation en direction du fond du corps **2** projetant ainsi le liquide à injecter au-travers de l'aiguille **9**. Le déplacement de l'embout **5** relativement au piston **3** libère les prolongements des pattes **13** de l'anneau de mémorisation **6**. Par conséquent, celui-ci se trouve décalé par rapport au piston **3** et aux pattes **13** et occupe la position représentée à la figure 4.

Il en résulte que, comme le montre la figure 5, si l'on veut pratiquer un nouveau remplissage, en exerçant une traction sur la tige **4**, les pattes **13**, libérées de l'emprise de l'anneau de mémorisation **6**, se déforment élastiquement et laissent échapper l'embout **5**, comme représenté en traits mixtes sur la figure 5.

Il s'ensuit donc que le piston **3** reste en place dans le corps de la seringue **2** et que l'embout **5** est désolidarisé totalement de l'ensemble piston/anneau sans pouvoir être remis dans la position qu'il occupait à la figure **2**.

Un second remplissage de la chambre intérieure **12** est alors impossible, ce qui rend la seringue **1** inutilisable et évite ainsi tout risque de contamination.

Dans cet exemple, la sécurité est encore améliorée du fait de l'inviolabilité de la liaison entre l'aiguille et son support.

Dans la forme d'exécution représentée à la figure 6, le piston **10** comporte un orifice central traversant **40**, dans lequel est destiné à être engagé un doigt **41** solidaire de l'embout **5**. L'engagement du doigt **41** dans l'orifice **40** assure une fermeture de celui-ci avec étanchéité. Après première utilisation de la seringue et désolidarisation de l'embout **5** et du piston **10**, l'orifice **40** est ouvert, de telle sorte qu'il n'est pas possible d'assurer le mouvement de retour du piston par injection de fluide sous pression à partir de l'aiguille. Ceci améliore encore le caractère unique de l'usage de cette seringue.

Comme il va de soi, l'invention ne se limite pas à la forme d'exécution de la seringue, décrite ci-dessus à titre d'exemple ; elle en embrasse, au contraire, toutes les variantes de réalisation telles que comprises dans les définitions données avec les revendications suivantes.

C'est ainsi que la seringue pourrait comporter un support d'aiguille classique tel que ceux existants actuellement constitués par un tube sur lequel s'emmanche le capuchon supportant la partie métallique de l'aiguille, que l'anneau de mémorisation pourrait être continu ou non. Dans ce dernier cas, il présenterait une fente lui conférant une certaine élasticité facilitant son introduction dans le corps de seringue ou que le prolongement du piston pourrait être constitué par un nombre différent de pattes ou par une jupe continue et déformable.

## Revendications

1. Seringue à usage unique, du type comprenant un corps cylindrique (2) creux à l'intérieur duquel est mobile un piston (3) délimitant, de manière étanche, avec l'extrémité du corps comportant un support (8) d'aiguille (9), une chambre intérieure (12) de volume variable, le piston (3) étant relié à l'extrémité intérieure (5) d'une tige de piston (4) permettant la manoeuvre de celui-ci, et des moyens (6,13) permettant, d'une part, de solidariser le piston (3) à l'extrémité intérieure (5) de la tige de piston (4) lors du premier remplissage de la chambre intérieure (12), et, d'autre part, de provoquer la désolidarisation du piston (3) et de l'extrémité intérieure (5) de la tige de piston (4) lors d'une éventuelle tentative de remplissage ultérieur de la chambre intérieure (12), caractérisée en ce que l'extrémité intérieure de la tige de piston (4) comporte un embout (5) d'axe correspondant à celui du corps (2) et présentant au moins une région tronconique (19) dont le plus grand diamètre est situé du côté du piston (3), cette région tronconique (19) étant disposée, au montage, dans un prolongement (16) creux du piston (3) tourné du côté de la tige (4), de manière à former un coin bloquant ce prolongement (16) contre un organe de mémorisation, constitué par un anneau (6) dont la périphérie est en appui contre la paroi (22) du corps cylindrique.

2. Seringue selon la revendication 2, caractérisée en ce que la région tronconique (19) de l'embout (5) de la tige (4) est prolongée, d'une part, par une partie cylindrique (18) fixée sur la tige de piston (4) et, d'autre part, du côté de son extrémité libre, par une partie tronconique convergente (20).

3. Seringue selon l'une quelconque des revendications 1 et 2, caractérisée en ce que le prolongement (16) creux du piston (3) est constitué par quatre pattes (13) s'étendant à partir de la périphérie de celui-ci, décalées angulairement de 90° les unes par rapport aux autres, comprenant, chacune, depuis le piston (3), une partie parallèle à l'axe de la seringue (1) et prolongée par une partie inclinée vers l'intérieur, les faces extérieure (14) et intérieure de chaque patte (13) étant destinées à venir en contact respectivement avec la face intérieure (21) de l'anneau de mémorisation (6) et la région tronconique (19) de l'embout (5).

4. Seringue selon la revendication 2 et la revendication 3, caractérisée en ce que la longueur de la partie convergente (20) de l'embout (5) est inférieure à celle de la partie de chaque patte du piston (3) parallèle à l'axe de la seringue (1).

5. Seringue selon l'une quelconque des revendications 1 à 4, caractérisée en ce que l'embout (5) est fixé à l'extrémité de la tige (4) par clipsage sur un têton (17) solidaire de celle-ci.

6. Seringue selon l'une quelconque des revendications 1 à 5, caractérisée en ce que le support (8) d'aiguille (9) situé à l'une des extrémités du corps (2) se présente sous la forme d'un tube (30) de diamètre inférieur à celui du corps (2) reliant la chambre intérieure (12) de la seringue (1) à l'extérieur et destiné à recevoir, sur sa surface extérieure, un capuchon (34) de forme complémentaire supportant la partie métallique creuse (33) de l'aiguille (9) à l'une de ses extrémités, le tube (30) étant disposé à l'intérieur d'une jupe cylindrique (31) creuse solidaire du corps (2) par l'une de ses extrémités et possèdant une longueur supérieure à celle du tube (30) de telle sorte que, lorsque le capuchon (34) est emmanché sur le tube, la jupe (31) enveloppe totalement ce capuchon (34).

7. Seringue selon la revendication 6, caractérisée en ce que le capuchon (34) est équipé d'un rebord annulaire (35) apte à s'engager, de façon irréversible, dans un logement (36) de forme complémentaire prévu sur la jupe (31) ou sur le tube (30).

8. Seringue selon l'une quelconque des revendications 1 à 7, caractérisée en ce que le piston (10) comprend au moins un orifice traversant (40), destiné à être obturé par un doigt (41) solidaire de l'embout (5), lorsque le piston et l'embout sont en position rapprochée.


**Patentansprüche**

1. Spritze zum einmaligen Gebrauch, beinhaltend einen zylindrischen hohlen Körper (2), in dessem Inneren ein Kolben (3) beweglich ist, der auf abgedichtete Weise mit dem einen Träger (8) für die Nadel (9) aufweisenden Ende des Körpers eine innere Kammer (12) variablen Volumens begrenzt, wobei der Kolben (3) mit dem inneren Ende (5) einer Kolbenstange (4) verbunden ist, die seine Betätigung ermöglicht und Mittel (6, 13) vorgesehen sind, die einerseits die Festsetzung des Kolbens (3) am inneren Ende (5) der Kolbenstange (4) bei der Erstfüllung der inneren Kammer (12) ermöglichen und andererseits die Loslösung des Kolbens (3) und des inneren Endes (5) der Kolbenstange (4) bei einem eventuellen Versuch einer späteren weiteren Füllung der inneren Kammer (12) hervorrufen, **dadurch gekennzeichnet,** daß das innere Ende der Kolbenstange (4) ein Ansatzstück (5) mit einer Achse entsprechend derjenigen des Körpers (2) trägt, das mindestens eine kegelstumpfförmige Zone (19) aufweist, deren größerer Durchmesser auf der Seite des Kolbens (3) liegt, wobei diese kegelstumpfförmige Zone (19) bei der Montage in einer hohlen Verlängerung (16) des Kolbens (3), die der Seite der Kolbenstange (4) zugewandt ist, angeordnet ist derart, daß sie einen Keil bildet, der diese Verlängerung (16) gegen ein Speicherorgan blockiert, das durch einen Ring (6) gebildet ist, dessen Peripherie gegen die Wand (22) des zylindrischen Körpers abgestützt ist.

2. Spritze nach Anspruch 2, dadurch gekennzeichnet, daß die kegelstumpfförmige Zone (19) des Ansatzstückes (5) der Stange (4) einerseits durch einen zylindrischen Abschnitt (10), der auf der Kolbenstange (4) befestigt ist, und andererseits auf der Seite seines freien Endes durch einen konvergierenden kegelstumpfförmigen Abschnitt (20) verlängert ist.

3. Spritze nach einem der Ansprüche 1 und 2, dadurch gekennzeichnet, daß die hohle Verlängerung (16) des Kolbens (3) von vier Klauen (13) gebildet ist, die sich ausgehend von der Kolbenperipherie erstrecken und winkelmäßig um 80° versetzt zueinander sind und die jede, ausgehend vom Kolben (3), einen Abschnitt parallel zur Achse der Spritze (1) aufweisen, der durch einen nach innen geneigten Abschnitt verlängert ist, wobei die äußeren Flächen (14) sowie die inneren Flächen jeder Klaue (13) dazu bestimmt sind, respektive mit der Innenfläche (21) des Speicherringes (6) und der kegelstumpfförmigen Zone (19) des Ansatzstückes (5) in Kontakt zu kommen.

4. Spritze nach Anspruch 2 und Anspruch 3, dadurch gekennzeichnet, daß die Länge des konvergierenden Abschnittes (20) des Ansatzstückes (5) geringer ist als diejenige des parallel zur Achse der Spritze (1) liegenden Abschnittes einer jeden Klaue des Kolbens (3).

5. Spritze nach einem der Ansprüche 1 - 4, dadurch gekennzeichnet, daß das Ansatzstück (5) am Ende der Stange (4) durch Verclipsung auf einer Ansatzspitze (17) festgesetzt ist, die fest mit der Stange verbunden ist.

6. Spritze nach einem der Ansprüche 1 - 5, dadurch gekennzeichnet, daß der Träger (8) der Nadel (9), der an dem einen Ende des Körpers (2) angeordnet ist, die Form eines Rohres (30) aufweist, mit einem Durchmesser kleiner als demjenigen des Körpers (2), das die Innenkammer (12) der Spritze (1) mit der Umgebung verbindet und dazu bestimmt ist, auf seiner Außenfläche eine Kappe (14) komplementärer Form aufzunehmen, die den metallischen hohlen Abschnitt (33) der Nadel (9) an seinem einen Ende trägt, wobei das Rohr (30) im Inneren eines zylindrischen hohlen Stutzens (31) angeordnet ist, der mit seinem einen Ende fest mit dem Körper (2) verbunden ist und eine Länge größer als diejenige des Rohres (3) aufweist derart, daß bei auf dem Rohr festgesetzter Kappe (34) der Stutzen (31) diese Kappe (34) vollständig umhüllt.

7. Spritze nach Anspruch 6, dadurch gekennzeichnet, daß die Kappe (34) mit einem ringförmigen Flansch (35) versehen ist, der geeignet ist, in unlösbarer Weise in eine Ausnehmung (36) komplementärer Form einzugreifen, die an dem Stutzen (31) oder auf dem Rohr (30) vorgesehen ist.

8. Spritze nach einem der Ansprüche 1 - 7, dadurch gekennzeichnet, daß der Kolben (10) mindestens eine Durchgangsöffnung (43) aufweist, die durch einen fest mit dem Ansatzstück (5) verbundenen Finger (41) verschließbar ist, wenn der Kolben und das Ansatzstück in der Annäherungslage sind.

## Claims

1. Syringe for single use, of the type comprising a hollow cylindrical body (2) inside which a piston (3) is movable, defining, sealingly, with the end of the body which includes a support (8) for a needle (9), an internal chamber (12) of variable volume, the piston (3) being connected to the inner end (5) of a piston stem (4) enabling the latter to be manoeuvred, and means (6, 13) making it possible, on the one hand, to connect the piston (3) to the inner end (5) of the piston stem (4) when the inner chamber (12) is first filled and, on the other hand, to bring about the disconnection of the piston (3) and the inner end (5) of the piston stem (4) during any subsequent attempt at filling the inner chamber (12), characterised in that the inner end of the piston stem (4) includes a joining piece (5) with an axis corresponding to that of the body (2) and having at least one tapered area (19), the largest diameter of which is situated on the piston (3) side, this tapered area (19) being disposed, at the time of assembly, in a hollow extension (16) of the piston (3) oriented towards the stem (4), so as to form a wedge locking this extension (16) against a memorising device, consisting of a ring (6) whose peripheiy bears against the wall (22) of the cylindrical body.

2. Syringe according to claim 2, characterised in that the tapered area (19) of the joining piece (5) on the stem (4) is extended on the one hand by a cylindrical part (18) fixed to the stem of the piston (4) and on the other hand, at its free end, by a converging tapered part (20).

3. Syringe according to either one of claims 1 and 2, characterised in that the hollow extension (16) of the piston (3) is formed by four lugs (13) extending from its periphery, offset angularly by 90° with respect to each other, each comprising, from the piston (3), a part parallel to the axis of the syringe (1) and extended by a-part sloping towards the inside, the external (14) and internal faces of each lug (13) being designed to come into contact respectively with the internal face (21) of the memorising ring (6) and the tapered area (19) of the joining piece (5).

4. Syringe according to claim 2 and claim 3, characterised in that the length of the converging part (20) of the connecting piece (5) is less than that of the part of each lug on the piston (3) parallel to the axis of the syringe (1).

5. Syringe according to any one of claims 1 to 4, characterised in that the joining piece (5) is fixed to the end of the stem (4) by snapping onto a stud (17) secured to the latter.

6. Syringe according to any one of claims 1 to 5, characterised in that the support (8) for the needle (9) situated at one end of the body (2) is in the form of a tube (30) with a diameter less than that of the body (2) connecting the inner chamber (12) of the syringe (1) to the outside and designed to be fitted, on its external surface, with a cap (34) with a complementary shape supporting the hollow metallic part (33) of the needle (9) at one of its ends, the tube (30) being disposed inside a hollow cylindrical skirt (31) secured to the body (2) by one of its ends and having a length greater than that of the tube (30) so that, when the cap (34) is fitted on the tube, the skirt (31) totally encloses this cap (34).

7. Syringe according to claim 6, characterised in that the cap (34) is provided with an annular rim (35) suitable for engaging, irreversibly, in a recess (36) with a complementary shape provided on this skirt (31) or on the tube (30).

8. Syringe according to any one of claims 1 to 7, characterised in that the piston (10) comprises at least one orifice (40) passing through it, designed to be closed off by a pin (41) fixed to the connecting piece (5), when the piston and connecting piece are in a position close together.

FIG.6

FIG.1

FIG.2

FIG.3

FIG.4

FIG.5